# EUROPÄISCHE PATENTSCHRIFT

(11) **EP 1 444 253 B1**
(45) Veröffentlichungstag und Bekanntmachung des Hinweises auf die Patenterteilung: **29.03.2006**
(21) Anmeldenummer: 02782765.8
(22) Anmeldetag: 08.11.2002
(51) Int. Cl.: C07K 14/00

(54) **PNA-KONJUGAT ZUR THERAPIE DER CHRONISCHEN MYELOISCHEN LEUKÄMIE (CML)**
PNA-CONJUGATES FOR TREATING CHRONIC MYELOID LEUKEMIA (CML)
CONJUGUES DE PAN POUR TRAITER LES LEUCEMIES MYELOIDES CHRONIQUES (CML)

(30) Priorität: 08.11.2001 DE 10154827
(43) Veröffentlichungstag der Anmeldung: 11.08.2004
(73) Patentinhaber: Deutsches Krebsforschungszentrum Stiftung des öffentlichen Rechts, 69120 Heidelberg (DE)
(72) Erfinder: BRAUN, Klaus, 69207 Sandhausen (DE); WALDECK, Waldemar, 69514 Laudenbach (DE); PIPKORN, Rüdiger, 69120 Heidelberg (DE); BRAUN, Isabell, 35091 Cölbe-Bürgeln (DE); DEBUS, Jürgen, 69121 Heidelberg (DE)
(74) Vertreter: Schüssler, Andrea
(86) Internationale Anmeldenummer: PCT/DE2002/004154
(87) Internationale Veröffentlichungsnummer: WO 2003/039438

(56) Entgegenhaltungen:
- US-B1- 6 312 956
- SZCZYLIK C ET AL: "SELECTIVE INHIBITION OF LEUKEMIA CELL PROLIFERATION BY BCR-ABL ANTISENSE OLIGODEOXYNUCLEOTIDES" SCIENCE, AMERICAN ASSOCIATION FOR THE ADVANCEMENT OF SCIENCE,, US, Bd. 253, 2. August 1991 (1991-08-02), Seiten 562-565, XP002027489 ISSN: 0036-8075
- CHALOIN L ET AL: "Design of carrier peptide-oligonucleotide conjugates with rapid membrane translocation and nuclear localization properties." BIOCHEMICAL AND BIOPHYSICAL RESEARCH COMMUNICATIONS, Bd. 243, Nr. 2, 13. Februar 1998 (1998-02-13), Seiten 601-608, XP002238939 ISSN: 0006-291X
- BRAUN KLAUS ET AL: "A biological transporter for the delivery of peptide nucleic acids (PNAs) to the nuclear compartment of living cells." JOURNAL OF MOLECULAR BIOLOGY, Bd. 318, Nr. 2, 2002, Seiten 237-243, XP002238940 26 April, 2002 ISSN: 0022-2836
- RAPOZZI VALENTINA ET AL: "Antiproliferative effect in chronic myeloid leukaemia cells by antisense peptide nucleic acids." NUCLEIC ACIDS RESEARCH, Bd. 30, Nr. 17, 1. September 2002 (2002-09-01), Seiten 3712-3721, XP002238941 September 1, 2002 ISSN: 0305-1048

## Beschreibung

Die vorliegende Erfindung betrifft Peptid-Nukleinsäure(PNA)-Konjugate, die die Expression des für CML charakteristischen Fusionsgens *bcr*/*abl* hemmen und zur Therapie der chronischen myeloischen Leukämie (CML) geeignet sind.

Die chronische myeloische Leukämie (CML) ist die häufigste myeloproliferative Erkrankung. An ihr konnte, erstmalig bei einer hämatologischen Erkrankung, eine charakteristische Chromosomenaberration gezeigt werden. Das pathophysiologisch relevante *bcr*/*ab1*-Fusionsgen entsteht i.d.R als Folge einer Translokation t(9;22) und ist auf dem gekürzten Chromosom 22, dem sogenannten Philadelphia-Chromosom (Ph), zu finden. Bei dem entsprechenden Genprodukt handelt es sich um ein Protein (p210) mit Tyrosinkinaseaktivität. Über 95% der CML-Patienten sind BCR/ABL-positiv. Die Bildung des Fusionsgens in hämatopoietischen Stammzellen ist der wesentliche Schritt zur Veränderung des Regulationsverhaltens der Stammzellen und führt zum charakteristischen hämatologischen und klinischen Bild der CML. Bei supprimiertem BCR-ABL-Protein sind diese Abnormitäten stark verringert. Dies hat zu unterschiedlichen Therapieansätzen geführt, die im wesentlichen auf eine spe ifische Eliminierung der BCR-ABL-Expression zielen.

Bezüglich der aktuellen Therapieansätze ist anzumerken, daß zur Zeit die allogene Stammzelltransplantation den alleinigen kurativen Therapieansatz darstellt. Diese Therapie weist jedoch eine hohe Toxizität auf und ist auf jüngere CML-Patienten mit passenden Spendern begrenzt. Konventionelle Strategien zur Behandlung der CML basieren auf einer Therapie mit Interferon-α und Hydroxyharnstoff - u.U. in Kombination mit Cytarabin. Diskutiert werden zur Zeit auch experimentelle Verfahren wie z.B. autologe Stammzelltransplantation. Es wird auch über einen günstigen Verlauf klinischer Studien berichtet, die auf einem cABL-spezifischen Inhibitor (STI 571) basieren. Allerdings scheint STI 571 mangels Spezifität auch mit weiteren Tyrosin-Kinasen außer dem BCR-ABL-Protein zu interagieren. Auch muß in diesem Zusammenhang noch beachtet werden, daß die primitiven CML-Progenitorzellen wohl weder BCR-ABL-mRNA noch p210 exprimieren und somit einer Therapie mit ST 571 wie auch der klassischen, gegen mRNA gerichteten Antisense-Therapie unzugänglich sind. Bezüglich der bisher durchgeführten Antisense-Studien ist noch festzuhalten, daß diese insofern unbefriedigend ausfielen, da mangels ausreichender biologischer Halbwertszeit der verwendeten Antisense-Oligonukleotide im Vergleich zu der Halbwertszeit der BCT-ABL mRNA diese unwirksam waren. Auch konnte bisher die Frage eines effizienten Heranbringens der Antisense-Oligonukleotide zum Zielort durch biologische Membranen nicht gelöst werden. Zudem erlitten sämtliche mit den vorstehend erwähnten bereits etablierten Therapien behandelte Patienten leukämische Relapse mit ungünstiger Prognose. Da diese Therapien somit keinen entscheidenden Durchbruch bei der CML-Behandlung erzielen konnten, ist die Erfolgsprognose z.Zt. ungünstig und verdeutlicht die Notwendigkeit für effektivere Therapien.

Somit liegt der Erfindung im wesentlichen das technische Problem zugrunde, Mittel bereitzustellen, die eine wirksame Therapie der chronischen myeloischen Leukämie (CML) erlauben.

Die Lösung dieses technischen Problems erfolgte durch die Bereitstellung der in den Patentansprüchen gekennzeichneten Ausführungsformen. Von den Erfindern wurde zum Erzielen der Lösung des technischen Problems ein Konjugat bzw. ein Konjugat-Gemisch entwickelt, das die folgenden Komponenten umfaßt: (a) einen Transportvermittler für die Zellmembran (P), (b) ein Adressprotein bzw. -peptid (AP) für den Import in den Zellkern, und (c) eine zu transportierende, mit dem Fusionsgen *bcr*/*abl* spezifisch hybridisierbare Peptid-Nukleinsäure (PNAs), die dessen Expression hemmt.

Diese modular aufgebauten Konjugate weisen zwei entscheidende Vorteile auf:
(a) Mittels der Komponenten P und AP wird ein effizienter und gerichteter Transport der PNA zu dem Zielort und somit eine Gentherapie ermöglicht. Diese Komponenten erlauben nicht nur einen schnellen und effektiven Transport der PNA durch Zellmembranen lebender Zellen ins Zytoplasma, sondern auch, nach zytoplasmatischer Aktivierung von Adresspeptidsequenzen, einen effizienten Transport in den Zellkern.
(b) Der Einsatz der protease- und nukleaseresistenten Peptid-Nukleinsäuren ("PNAs"), bei denen es sich um Oligonukleotid-Derivate handelt, bei denen das Zuckerphosphat-Rückgrat bevorzugt durch Ethyl-Amin verbundene α-Amino-Ethyl-Glycin-Einheiten substituiert ist, erlaubt aufgrund ihrer physikochemischen Eigenschaften unter physiologischen Bedingungen eine stabile und effiziente Blockierung der Transkription der gewünschten Gene. Mit diesen PNAs wird eine auf dem Antisense-Prinzip basierende Anti-Gen-Strategie verfolgt, bei der jedoch nicht die mRNA sondern das Gen selbst das Ziel ist. Dabei hybridisieren die PNAs über die Bildung einer Triple-Helix an die Ziel-DNA und können so die Synthese des Fusionsproteins p210 hemmen. Aufgrund der Spezifität der erfindungsgemäßen PNA-Konjugate ist es möglich, individuell rekombinante Fusions-*bcr*/*abl*-DNA von unveränderter, nicht fusionierter *bcr*- und *abl*-DNA zu unterscheiden.

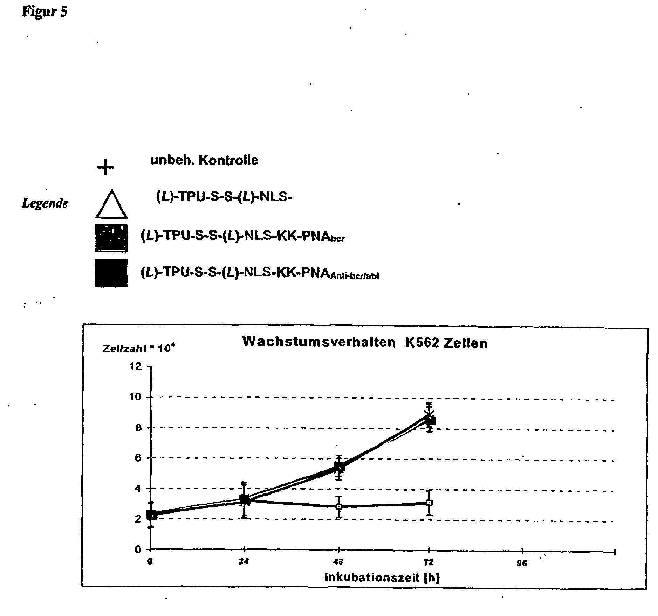

Zusammengefaßt kann festgestellt werden, daß die erfindungsgemäßen Konjugate eine neue Art von Pharmaka darstellen, die effektiv an den Zielort transportiert werden und aufgrund ihrer hohen Stabilität und ausgezeichneten Spezifität eine stabile und effiziente Transkriptionskontrolle von *bcr*/*abl* auf genomischer Ebene ermöglichen. Die erfindungsgemäßen PNA-Konjugate sollten selbst bei äußerst niedrigen Verabreichungsdosen (unter 100 pM Endkonzentration) wirksam sein, wodurch das Auftreten unerwünschter Nebenwirkungen verhindert oder zumindest stark verringert wird. Die Spezifität der erfindungsgemäßen PNA-Konjugate wird dadurch erreicht, daß Rekombinations-"Hotspots" mit etablierten Verfahren wie PCR, nachfolgender Sequenzierung und Identifizierung mittels Biocomputing-Verfahren charakterisiert werden und darauf aufbauend die PNA-Sequenzen der erfindungsgemäßen Konjugate für ein individuelles Wirkstoff-Design entworfen werden. Dies konnte anhand des Proliferationsstops der Tumor-Zellinie K562 (Phpositiv) (Grosveld et al., Molecular and Cellular Biology 6 (2) (1986), 607-616) experimentell in dem nachstehenden Beispiel 3 (s.a. Figur 5) nachgewiesen werden.

Somit betrifft die vorliegende Erfindung ein Konjugat zur spezifischen Hemmung der Expression eines *bcr*/*abl-*Fusionsgens, wobei das Konjugat die folgenden Komponenten aufweist: (a) einen Transportvermittler für die Zellmembran, (b) ein Adressprotein bzw. -peptid für den Import in Zellkompertimente, vorzugsweise den Zellkern und (c) eine mit dem Fusionsgen *bcr*/*abl* spezifisch hybridisierbare Peptid-Nukleinsäure (PNA), die dessen Expression hemmt. Bezüglich Verfahren zur Herstellung der einzelnen Komponenten der Konjugate und zu deren Verknüpfung wird auf die deutsche Patentanmeldung Nr. 199 33 492.7 verwiesen. Die Synthese von PNAs ist dem Fachmann bekannt und z.B. auch in Nielsen et al., Science 254 (1991), 1497-1500, beschrieben.

Der Aufbau des erfindungsgemäßen Konjugats ist vorzugsweise: P-AP-PNA_{*bcr*/*abl*-spezifisch}, noch mehr bevorzugt P-S-S-AP-Spacer-PNA_{*bcr*/*abl-*spezifisch}, wobei -S-S- einer Disulfidbrücke entspricht.

Den Transportvermittler für die Zellmembran stellt vorzugsweise ein Peptid bzw. Protein dar, das die Plasmamembran überwinden kann. Die Länge dieses Peptids bzw. Proteins unterliegt keiner Beschränkung, solange es die obige Eigenschaft aufweist. Transportvermittler sind beispielsweise viralen, bakteriellen oder humanen Ursprungs und stammen vorzugsweise aus der Penetratin-Familie (Derossi et al., Trends Cell Biol. 8 (1988), S. 84-87), sind Tränsportan bzw. Teile davon (Pooga et al., The Faseb Journal 12 (1998), S. 68 ff.), das Transmembranpeptid pAntp (43-58), TPU^{Eco}, bevorzugte Aminosäuresequenz: H₂N-MTRQTFWHRIKH-COOH), TPU^{HIV-1/TAT}, bevorzugte Aminosäuresequenz: H₂N-YGRKKRRQRRR-COOH, oder TPU^{Hum}, bevorzugte Aminosäuresequenz: H₂N-KMTRQTWWHRIKHKC-(Cys-CO-NH₂)-(SH)-CONH₂, H₂N-MTRQTFWHRIKHKC-(Cys-CO-NH₂)-(SH)-CONH₂ oder H₂N-KHKIRHWFTQRTMC-(Cys-CO-NH₂)-(SH)-CONH₂ ist.

Hergestellt wird der ausgewählte Transportvermittler auf biologischem Weg (Reinigung natürlicher Transportvermittlerproteine oder Klonierung und Expression der Sequenz in einem eukaryotischen oder prokaryotischen Expressionssystem), bevorzugt aber auf synthetischem Weg, z.B. nach dem Merrifield-Verfahren (Merrifield, J. Am. Chem. Soc. 85 (1963), 2149).

Die Auswahl des Adressproteins bzw. -peptids kann der Fachmann anhand der bekannten Aminosäuresequenzen für den Import in den Zellkern steuernde Peptide bzw. Polypeptide auswählen. Prinzipiell unterliegt die Länge dieses Adresspeptids bzw. -proteins keiner Beschränkung, solange es die Eigenschaft aufweist, einen zellkernspezifischen Transport zu gewährleisten. Für die Einbringung der PNAs werden im allgemeinen Adressproteine bzw. Adresspeptide ausgewählt, die ein zellkernspezifisches Erkennungssignal enthalten und dadurch die PNAs in den Zellkern dirigieren. Grundsätzlich ist für den Transport in den Zellkern die reine Adressequenz ausreichend. Es können aber auch Adressproteine/Adresspeptide ausgewählt werden, die über eine zytoplasmatische Peptidasespaltstelle verfügen. Diese Spaltstelle liegt im günstigsten Fall innerhalb der Signalsequenz, kann aber auch an diese durch zusätzliche Aminosäuren angefügt werden, um nach Erreichen des Zytoplasmas das Abspalten der Adressequenz sicherzustellen. Hergestellt wird die ausgewählte "AP"-Sequenz auf biologischem Weg (Reinigung natürlicher Transportvermittlerproteine oder Klonierung und Expression der Sequenz in einem eukaryontischen oder prokaryontischen Expressionssystem), bevorzugt aber auf synthetischem Weg, z.B. nach dem Merrifield-Verfahren (Merrifield, J. Am. Chem. Soc. 85 (1963), 2149). Beispiele für geeignete Adressproteine bzw. -peptide sind: (a) -Pro-Pro-Lys-Lys-Lys-Arg-Lys-Val und (b) H₃N⁺-Pro-Lys-Lys-Lys-Arg-Lys-Val- (= "Nuclear localisation sequence" (NLS) aus SV40-T-Antigen).

Weiter kann das Konjugat ggf. einen Spacer (vorstehend mit SP abgekürzt) enthalten, der sich vorzugsweise zwischen dem Adressprotein/-peptid und der zu transportierenden Peptid-Nukleinsäure (PNA) befindet. Er kann aber zusätzlich oder alternativ auch zwischen dem Transportvermittler und dem Adressprotein vorliegen. Der Spacer dient dazu, ggf. vorhandene sterische Wechselwirkungen zwischen den Komponenten aufzuheben bzw. günstig zu beeinflussen. Der Spacer kann beispielsweise ausgewählt sein aus Polylysin, Polyethylenglykol (PEG), Derivate der Poly-Methacrylsäure oder Polyvinylpyrrolidon (PVP).

Zwischen dem Transportvermittler und dem Adressprotein/-peptid befindet sich vorzugsweise eine Redoxspaltstelle, z.B. -Cystein-S-S-Cystein-O-N-H-. Die zwischen Transportvermittler und Adressprotein entstehende Bindung ist eine Redoxkopplung (schonende zellimmanente Verknüpfung mittels DMSO; Rietsch und Beckwith, Annu. Rev. Gent 32 (1998), 163-84):

Cystein-SH SH-Cystein -----> Cystin-S-S-Cystin

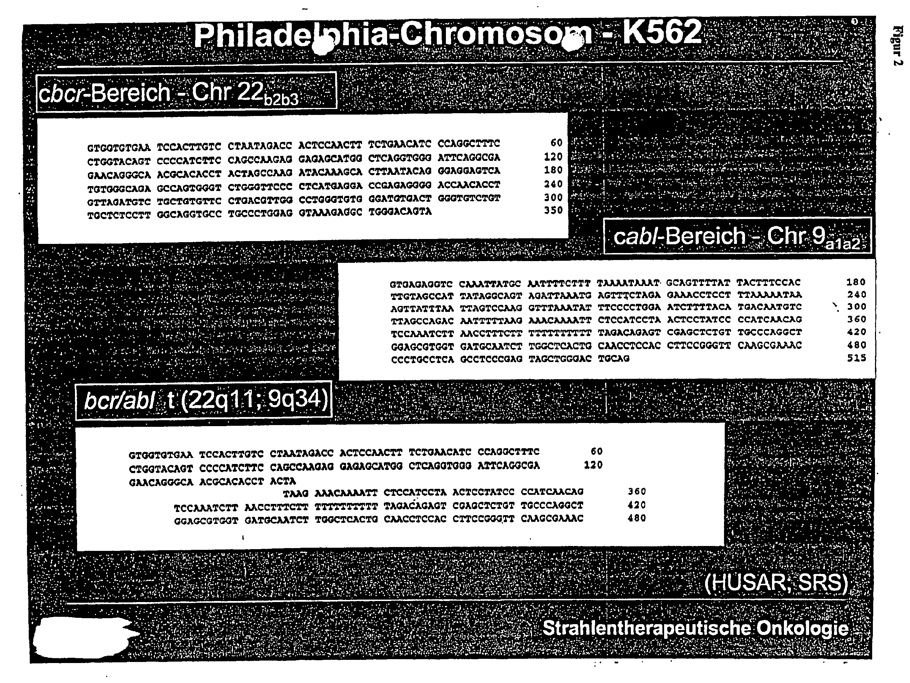

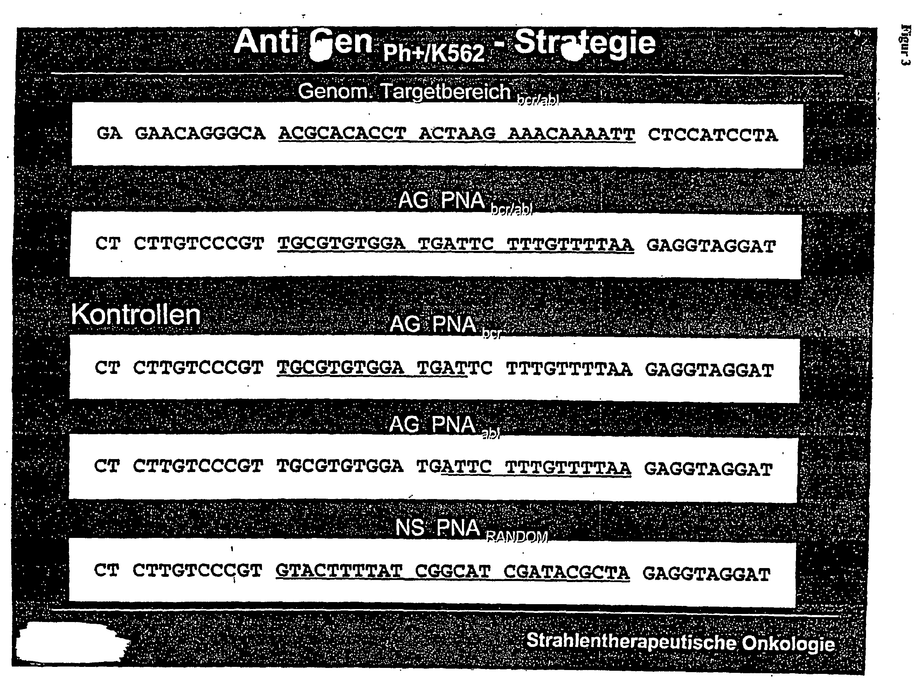

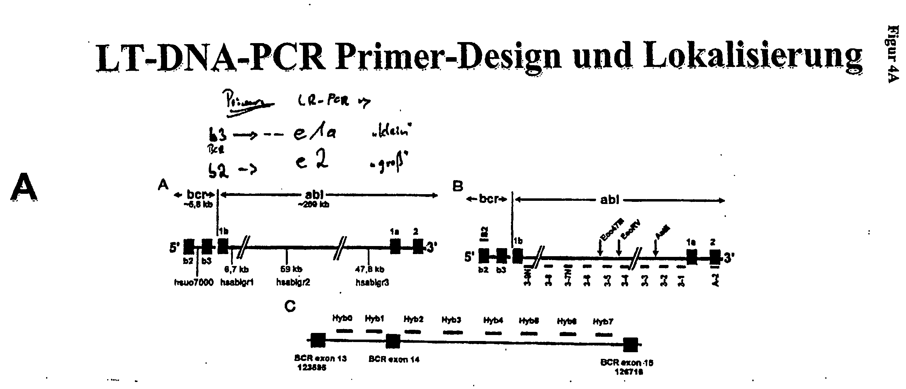

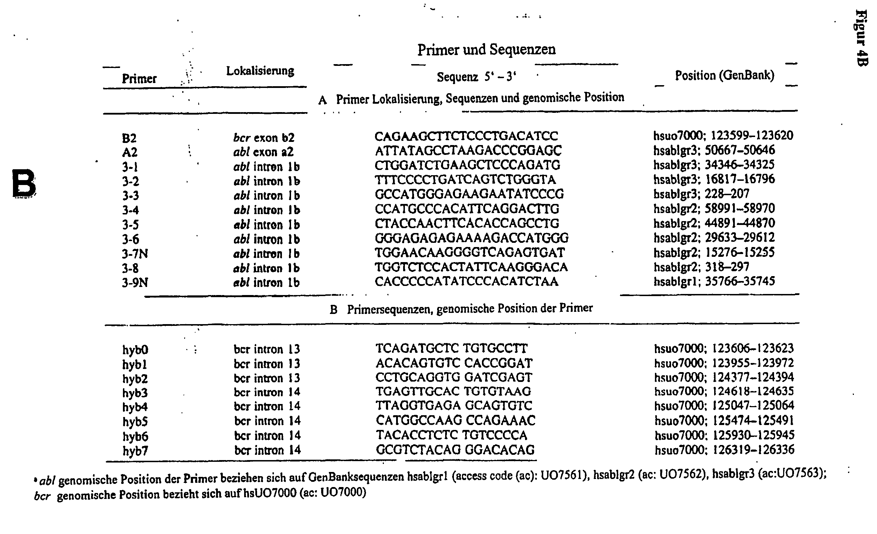

Die Peptid-Nukleinsäure (PNA) der erfindungsgemäßen Konjugate erlaubt die spezifische Hemmung der Transkription des *bcr*/*abl*-Fusionsgens unter Vermeidung der Hemmung der nativen, nicht-rekombinierten Gene *bcr* bzw. *abl* dadurch, daß sie mit einem Bereich des Fusionsgens hybridisiert, der transkribiert wird und den Rekombinationspunkt enthält. Geeignete Bereiche können vom Fachmann bestimmt werden, z.B. mittels des in dem nachstehenden Beispiel 2 beschriebenen Verfahrens, das schließlich zur Etablierung einer DNA-Bibliothek von Ph-Chromosom-positiven Proben mit anschließender Identifizierung individueller Rekombinations-Loci führen kann. Es können auch individuelle, auf den Patienten spezifisch abgestimmte Konjugate dadurch hergestellt werden, daß von diesem CML-Zellen entnommen und kultiviert werden, anschließend der Rekombinationspunkt z.B. anhand des in Figur 3 gezeigten molekularen Wirkorts an der genomischen DNA und mittels den in Figur 4 aufgelisteten Primern und der in Beispiel 2 beschriebenen LT-PCR und Sequenzierung bestimmt und die entsprechenden passenden PNA-Konjugate entworfen werden, d.h. PNA-Konjugate mit Sequenzen, die spezifisch nur an den *bcr*/*abl*-Rekombinationspunkt hybridisieren. Für die LT-PCR sollten vorteilhafterweise Primer mit einer Länge im Bereich von 25 bp, eine Anlagerungstemperatur bei 68 bis 69°C, ein GC-Gehalt der Primer von etwa 55% und eine Primer-Konzentration von etwa 20 pmol pro PCR-Ansatz gewählt werden. Vorzugsweise weisen die Peptid-Nukleinsäuren eine Länge von mindestens 20 Basen auf, besonders bevorzugt sind Peptid-Nukleinsäuren mit einer Länge von mindestens 23 Basen. Die Peptid-Nukleinsäure kann ggf. auch markiert sein, z.B. radioaktiv, mit einem Farbstoff, mit Biotin/Avidin usw.

Die Synthese der Konjugatbestandteile P und AP erfolgt vorzugsweise synthetisch nach dem Merrifield-Verfahren (Merrifield, J. Am. Chem. Soc. 85 (1963), 2149). Die Ankopplung der anderen Bestandteile (z.B. Spacer und/oder PNA) daran erfolgt durch kovalente chemische Bindung. Die Einfügung der Redoxspaltstelle zwischen P und AP erfolgt auf chemischen Wege durch die oben erwähnte Redoxkopplung. Auch zwischen einem ggf. vorhandenen Spacer und der PNA bzw. dem Adressprotein und der PNA liegt eine kovalente Bindung vor, bevorzugt eine Säureamid-Bindung. Mögliche Alternativen sind Ether- oder Ester-Bindungen, je nach den in der zu konjugierenden Substanz vorhandenen funktionellen Gruppe(n).

In einer besonders bevorzugten Ausführungsform des erfindungsgemäßen Konjugats umfaßt die Peptid-Nukleinsäure (PNA) folgende Sequenz: TGC GTG TGG ATG ATT CTT TGT TTT AA (Orientierung: N-Terminus/Sequenz/C-Terminus).

Die vorliegende Erfindung betrifft schließlich auch ein ein erfindungsgemäßes Konjugat, gegebenenfalls zusammen mit einem geeigneten Träger, enthaltendes Arzneimittel sowie dessen Verwendung zur Therapie von CML. Ein bevorzugter Verabreichungsweg ist eine intravenöse verabreichung.

Die Erfindung wird weiter anhand der nachfolgenden Figuren erläutert.

### Legenden zu den Figuren:

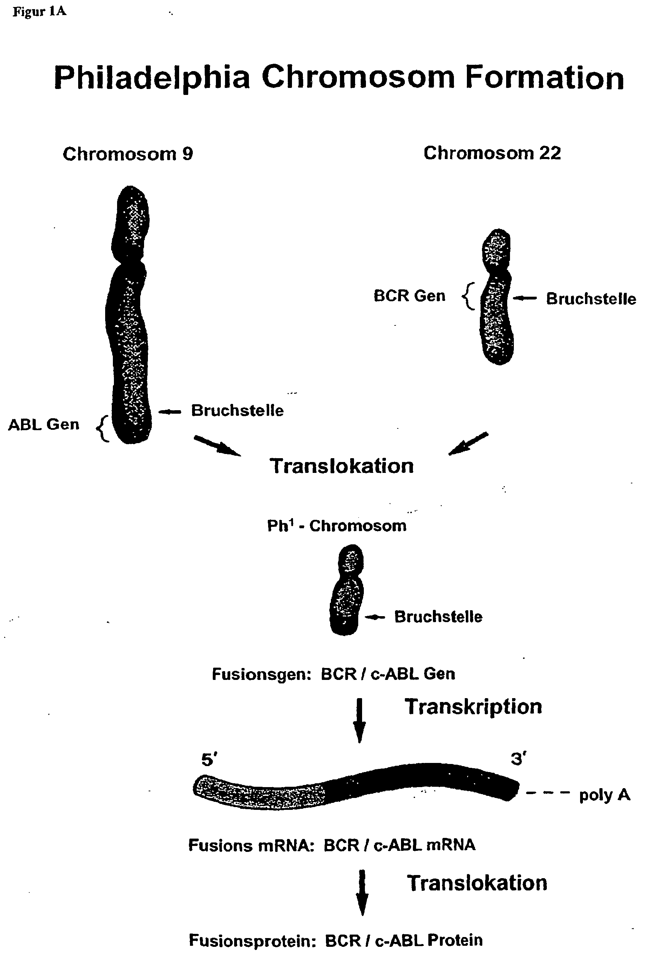

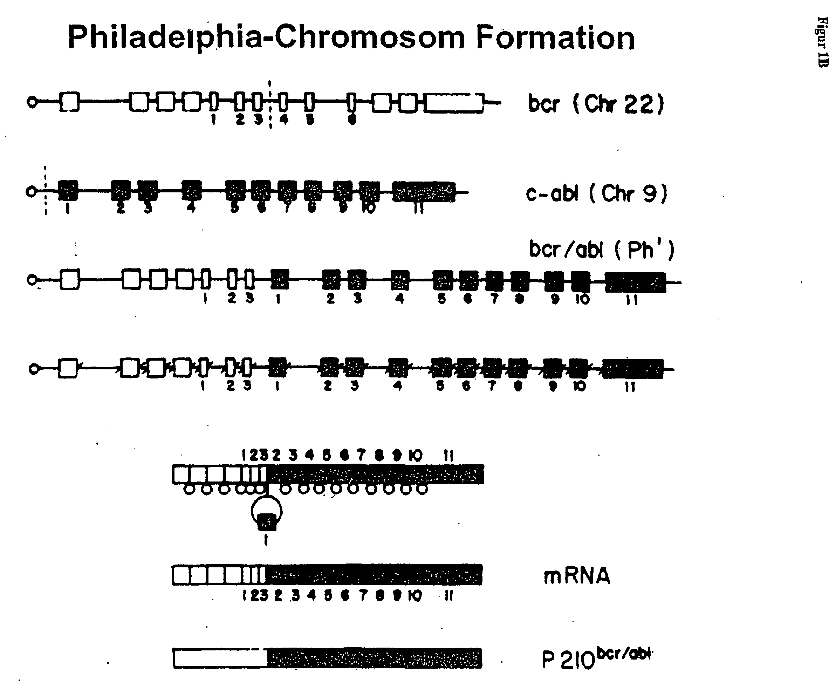

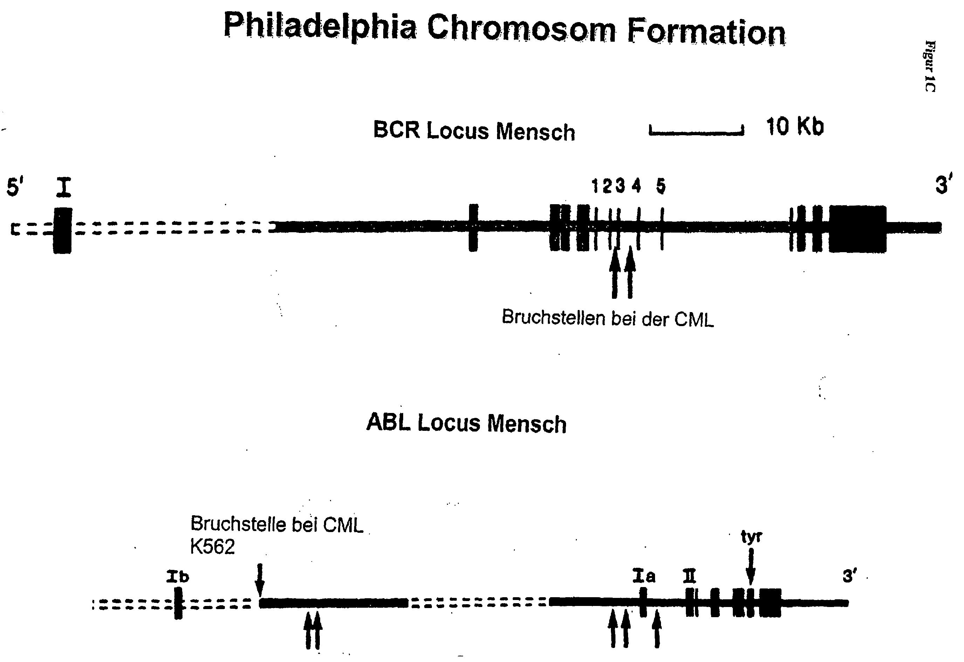

- **Figur 1:**: **Schematische Darstellung der Bildung des Philadelphia-Chromosoms durch Translokation zwischen Chromosom 9 und 22**
(A) Überblick
(B) Schematische Darstellung der Exon/Intron-Struktur des *bcr*/*abl*-Fusionsgens
(C) Genauere Darstellung der Bruchstellen innerhalb des bcr-Gens und des *abl*-Gens
- **Figur 2:**: **Darstellung des genomischen Wirkorts im Bereich des Rekombinationslocus des "Philadelphia-Chromosoms" am Beispiel der Zellinie K562**
oberer Bereich: DNA-Sequenzen des ursprünglichen bcr-Gens auf Chromosom 22
mittlerer Bereich: DNA-Sequenzen des ursprünglichen *abl*-Gens auf Chromosom 9
unterer Bereich: DNA-Sequenzen um die Rekombinationsstelle zwischen *bcr*-Gen und *abl*-Gen
- **Figur 3:**: **Darstellung der DNA-Sequenzen des genomischen Zielbereichs der K562-DNA für eine PNA-Konjugatstrategie**
Gezeigt ist
(A) der Zielbereich der genomischen *bcr*/*abl*-DNA der Zellinie K562
(B) die für eine Tränskriptionshemmung verwendete entsprechende Sequenz eines PNA-Konjugats und
(C) verschiedene Sequenzen für Kontroll-PNA-Konjugate.
- **Figur 4:**: **LT-DNA-PCR-Primerdesign und Lokalisierung**
(A) Schematische Darstellung der Lokalisation der verschiedenen Primer innerhalb des *bcr*/*abl-*Fusionsgens
(B) Darstellung der Sequenzen der verschiedenen Primer und deren Position (Angegeben sind die "rückwärts"-Primer.)
- **Figur 5:**: **Wachstumsverhalten von K562 Zellen nach Verabreichung verschiedener Konjugate**
Dargestellt ist die Zellzahlveränderung auf einer Zeitachse. Die Behandlung erfolgte über die gesamte experimentelle Dauer. Endkonzentration: 100 nM. Dunkelblaue Linie: AS-Konjugat; magenta: Zufallssequenz-Konjugat; gelb: AS-unligiert; blau: unbehandelte Kontrolle.
- **Figur 6:**: **Apoptoseverhalten von K562 Zellen nach Verabreichung verschiedener Konjugate**
Dargestellt ist die Zellzahlveränderung auf einer Zeitachse. Die Behandlung erfolgte über 24 h und anschließendem PNA-Entzug. Endkonzentration: 100 nM. Dunkelblaue Linie: AS-Konjugat; magenta: Zufallssequenz-Konjugat; gelb: AS-unligiert; blau: unbehandelte Kontrolle.

Die Erfindung wird weiter anhand der nachfolgenden Beispiele beschrieben.

### Beispiel 1

### Allgemeine Verfahren

### (A) Peptidsynthese

Peptidnukleinsäure (PNA) imitiert eine DNA und wurde ursprünglich als Reagens zur sequenzspezifischen Erkennung doppelsträngiger DNA über eine konventionelle Tripelhelix-Bildung entwickelt. Für die Festphasensynthese wurde die Fmoc-Strategie mittels einem vollautomatisierten Synthesegeräts (Syro II, Firma Multisyntech, Witten, Deutschland verwendet. Die Synthese wurde an einem 0,05 mmol Fmoc-AS-Polystyrenharz (1% quervernetzt) durchgeführt. Als Kopplungsreagenz wurde 2-(1H-Benzotriazol-1-yl)-1,1,3,3-Tetramethyluroniumhexafluorphosphat (HBTU) verwendet. Die Seitenketten schützenden Gruppen waren Lys(Boc), Asp(Obut), Ser(But), Cys(Trt) und Asn(Trt). Das geschützte Peptidylharz wurde mit 20% Piperidin in Dimethylformamid behandelt. Die Spaltung und Abspaltung der Schutzgruppen wurde durch Behandlung mit 90% Trifluoressigsäure, 5% Äthandithiol, 2,5% Thioanisol und 2,5% Phenol (Vol./Vol./Vol.) für 2,5 Stunden bei Raumtemperatur erzielt. Alle Produkte wurden in Äther präzipitiert und über präparative HPLC (Shimazu LC-8A, Shimazu, Duisburg, Deutschland) auf einer YMC ODS-A 7A S-7 µm Umkehrphasen-HPLC-Säule (20 x 250 mm) unter Verwendung von 0,1% Trifluoressigsäure in Wasser (A) und 60% Acetonitril in Wasser (B) als Elutionsmittel gereinigt. Die Peptide wurde mit einem linearen Gradienten von 25% B bis 60% B innerhalb von 40 Min. bei einer Durchflußrate von 10 ml/Min. eluiert. Die dem gereinigten Konjugat entsprechenden Fraktionen wurden lyophilisiert. Sequenzen von Einzelmolekülen sowie das vollständige bimodulare Konstrukt wurden über analytische HPLC (Shimadzu LC-10) und Laser-Desorptionsmassenspektroskopie (Vision 2000, Finnigan MAT, San Jose, CA, USA) wie nachstehend angegeben charakterisiert. Das Zufalls-PNA-Konstrukt wurde mit einem identischen Trägerkonjugat hergestellt.

### Peptidreinigung:

Gradient: analyt. 5%→ 80% (in einem Zeitraum von 35 Min.); präparativ: 5%→ 80% (in einem Zeitraum von 40 Min.);
Reinheit: >90%.

Die Verknüpfung des Peptids an die weiteren Bestandteile des Konjugats erfolgte nach üblichen Verfahren (siehe vorstehende Beschreibung).

### (B) Messungen des Zellwachstums

Die Messungen des Zellwachstums erfolgtem mit einem "Light-Cycler"-Gerät (Roche, Basel, Schweiz). Die Zellzahl wurde mit einem "Coulter Counter"-Gerät gemessen

### (C) Durchflußzytometrie

Die Auswirkung der PNA-Konjugate auf die Lebensfähigkeit der K-562 Zellen und die Zellzyklusverteilung wurden über Durchflußzytomterie bestimmt. Die Durchflußzytomterie-Analysen wurden mit einem "PAS II"-Durehflußzytometer (Partec, Münster, Deutschland) durchgeführt, der mit einer 100 Watt Quecksilberdampflampe ausgerüstet war und Filterkombinationen für mit 2,4-Diamidino-2-Phenylindol (DAPI) gefärbten Zellen. Die Zellen wurden von nativen Proben bei Raumtemperatur unter leichtem Schütteln mit 2,1% Zitronensäure / 0,5% Tween® 20 gemäß dem Verfahren von de Villiers et al., Gynecol.Oncol. 44 (1992), 33-39, mit leichten Modifikationen isoliert. Für die Färbung der Zellsuspensionen wurde DAPI enthaltender Phosphatpuffer (7,2 g Na₂HPO₄ x 2 H₂O in 100 ml H₂O), pH-Wert 8,0) verwendet. Jedes Histogramm repräsentiert 30.000 bis 100.000 Zellen zur Messung des DNA-Index und des Zellzyklus. Zur Analyse der Histogramme wurde das "Multicycle"-Programm von Phoenix Flow Systems, San Diego, CA, verwendet. Die Lebensfähigkeit der Zellen wurde über ein neues Durchflußzytometrieverfahren bestimmt. Dazu wurden die Zellen mit den PNA-Konjugaten (100 pM) 24 bzw. 48 Stunden inkubiert. Als Kontrolle dienten unbehandelte Zellen. Die Analysen wurden in einem "FACS Calibur"-Durchflußzytometer (Becton Dickinson Cytometrie Systems, San Jose, CA) unter Verwendung der Vorwärts- und Seitwärtsstreuung durchgeführt und die relativen Fluoreszenzintensitäten der mit Propidiumjodid gefärbten nativen Zellen auf dem F2-Kanal bei lögarithmischer Gradeinteilung gemessen. Tote Zellen sind für Propidiumdijodid positiv und rot gefärbt, lebende Zellen bleiben ungefärbt.

### Beispiel 2

### Identifizierung individueller bcr/abl-Rekombinationsloci über LT-DNA-PCR und Sequenzierung

Obwohl die Mehrzahl der CML-Progenitorzellen den DNA-Rekombinationslokus besitzen, exprimieren nicht alle die *bcr*/*abl*-Hybrid-mRNA bzw. das Genprodukt p210. Diese Zellen wurden mit bisherigen Diagnosemethoden nicht erfaßt. Mit der nachstehend beschriebenen Strategie kann dieses Problem dadurch umgangen werden, daß die Rekombinationsstelle auf DNA-Ebene charakterisiert wird. Die chromosomale Bruchstelle liegt in einem Bereich von über 200 kbp und erfordert deshalb eine LT-DNA-PCR (Long Terminal DNA Polymerase Chain Reaction). Die nachfolgend beschriebenen spezifischen Primer ermöglichen eine Charakterisierung individueller Rekombinationsloci unabhängig von der mRNA-Expression und p210-Translation.

### (A) Probenentnahme

Es wurde peripheres Blut von 970 Patienten und 10 gesunden Kontrollpersonen verwendet. Weitere Kontrollen waren die Ph⁺-Zellinie K-562. Dabei wurde differenziert in: CT-, INF-, STI-571-behandelt, unbehandelt, Stadium.

### (B) DNA-Isolierung

DNA wurde nach Herstellerangaben mit dem "Qiagen Blood and Cell Cultur DNA Midi Kit" (Qiagen GmbH, Hilden, Deutschland) aus Blut isoliert. Dabei wurden aus jeweils etwa 10⁷ Zellen rund 100 ug genomische DNA enthalten. Diese wurde in Tris-EDTA-Puffer resuspendiert und bei -20°C eingefroren.

### (C) LT-DNA-PCR Primerdesign

Es wurde von den BCR- und Abl-Exons ausgegangen und dann schrittweise nach etwa 40 kbp in der genomischen Datenbank "HUSAR" nach passenden "rückwärts"-Primern gesucht. Für die einleitende PCR wurde ein Primerpaar verwendet, das in Exon b2 von *bcr* und exon a2 in *abl* hybridisiert, für die eigentliche LT-DNA-PCR Primer die in Exon 15 von *bcr* und Exon 2 von *abl* hybridisieren ( siehe Figur 4B). Die abl-Lokalisierung ist auf die GenBank-Sequenz (AC: UO7561) bezogen, wobei "ABL-PK" konstanter Primer in ABL-Exon 2 bedeutet. Die bcr-Lokalisierung ist auf die GenBank-Sequenz (U07000) bezogen, wobei "BCR-PK" konstanter Primer in BCR-Exon 15 bedeutet. Dabei werden PCR-Produkte im Bereich von etwa 50 kbp erhalten. Für die LT-PCR wurde ein Kit verwendet, der die "Advantage© Genomic"-Polymerase (Clontech Laboratories, Palo Alto, CA, USA) enthält, mit der PCR-Produkte bis etwa 40 kpb erhalten werden können, was große Schritte bei der Suche in genomischen Datenbanken erlaubt und somit eine ideale Basis für "Genomic Walking" darstellt. Die LT-PCR-Reaktionsansatz enthielt: etwa 20 ng genomische DNA, 2 µl dNTP-Mix (10 mM), 0,1 E/µl Polymerase, 1,5 mM MgCl₂, 20 pmol Primer (gemäß Figur 4B), Gesamtanzahl der Zyklen: 25 - 30. Die amplifizierten Produkte wurden auf ein 1,4% Agarosegel aufgetragen und die DNA aus den entsprechenden Banden über übliche Verfahren isoliert und sequenziert.

### Beispiel 3

### Hemmung der Proliferation der Tumorzellinie K562 nach Verabreichung der erfindungsgemäßen PNA-Konjugate

Für diese Untersuchungen wurde ein Konjugat mit folgender Struktur verwendet:
Transportpeptid (Penetratin) -Disulfidbrucke-NLS-KK_{(PITC)} - PNA^{BCR/Abl} (Sequenz von PNA^{BCR/Abl}: H₂N-TGC GTG TGG ATG ATT CTT TGT TTT AA-COOH). Die PNA-Sequenz des Zufallskonjugats ist aus Figur 3 ersichtlich.

### (A) Experimentelle Vorgehensweise

Die Aufreinigung des Konjugats erfolgte über analytische HPLC. Das Konjugat wurde als Stammlösung in 0,9% NaCl-Lösung (steril) angesetzt. Die K562 Zellen (frische Suspension) wurden über einen Zeitraum von 24 h in 10 ml-Falcon-Kulturflaschen (flacher Boden) in RPI 1649-Medium (Gibco) kultiviert (37°C, 5% CO₂). Vor Entfernen des RPMI-Mediums erfolgte die Behandlung der Zellen mit dem Konjugat nach folgendem Protokoll:
Ansatz 1 (Proliferation) ("Couter Counter"-Verfahren): 50 µl Konjugat-Stammlösung, 4950 µl Zellsuspension (K562, ca. 1000 Zellen pro ml).
Ansatz 2 (CMLS): 102,4 µg (Substanz) = 4,5 ml Gesamtvolumen (RPMI-Medium); Volumen-Glasträger mit 8 Kammern (300 ml pro Kammer).

Hierzu wurden für die Zeitmeßpunkte 3 Falcon-Flaschen mit jeweils 5 ml vorbereiteten Zellsuspension wie folgt behandelt: Das RPMI-Medium wurde entfernt, durch das gleiche Volumen des Gemischs gemäß Ansatz 2 ersetzt und bei 37°C in einer 5% CO₂-Atmosphäre 1 Stunde inkubiert. Die Kontrolle der Genexpression erfolgte dann anschließend nach Temperatur bei 37°C (Kontrolle). Die Lokalisation und das Erreichen des Zielorts wurden parallel mittels CLSM ermittelt. Dazu wurden die Zellen über eine Stunde vor der Messung auf 8-Kammerobjektträgern mit ZellTak® adhäriert. Das Medium wurde entfernt und zweimal mit farbstofffreiem RPMI-Medium gewaschen. Nach Einbetten mit Mowiol® wurde die FITC-Fluoreszenz via CLSM direkt bestimmt. Analog: Excitation 488 nm, Emission: 520 nm. Die Co-Lokalisation des NLS-Teilkonjugats wurde durch Zusatz von Lysotrek® bestimmt. Die Kernfärbung (DAPI) wurde über Vectashield®-Behandlung (Alexis Biochemicals, Grünberg, Deutschland) anstelle einer Mowiol®-Behandlung durchgeführt und mittels UV-Laser bestimmt.

### (B) Wachstumsverhalten von K562-Zellen (siehe Figur 5)

Die Behandlung von Proben und Kontrollen war identisch. Die finalen Wirkstoffkonzentrationen im RPMI-1640-Medium (farblos) betrugen 100 nM. Die Inkubationszeiten entsprachen der Experimentdauer. Es zeigte sich, daß weder die Konjugat-Zufallssequenz-PNA (magenta) noch die Anti-Gen-PNA ohne Transporter (gelb) einen Unterschied zur unbehandelten Kontrolle hinsichtlich des Wachstumsverhaltens zeigten. Nach 72 Stunden waren die Zellzahlen der Kontrollkulturen wegen chaotischem Wachstumsverhalten nicht mehr bestimmbar. Die mit Antisense-NLS-Penetratin (dunkelblau) behandelten K562-Tumorzellen zeigten eine Stagnation der Zellvermehrung (Stabilisierung der Zellzahl 24 Stunden nach Behandlung). Die Zellen zeigten außerdem im Gegensatz zu den Kontrollen einen vitalen Phänotyp. Mit Konjugaten, die sich hinsichtlich des Transportvermittlers unterschieden (bakteriell (TPU^{ECO}), bevorzugte Aminosäuresequenz: H₂N-MTRQTFWHRIKH-COOH), (viral (TPU^{HIV-1/TAT}), bevorzugte Aminosäuresequenz: H₂N-YGRKKRRQRRR-COOH, human (TPU^{Hum})), konnten ähnliche Ergebnisse erzielt werden.

### Beispiel 4

### Apoptoseverhalten der Tumorzellinie K562 nach Verabreichung der erfindungsgemäßen PNA-Konjugate

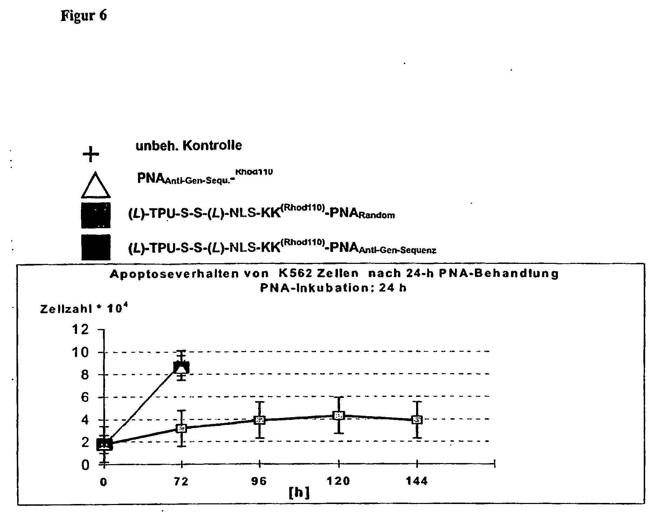

Es wurde die Frage hinsichtlich des Apoptoseverhaltens nach Verabreichung eines erfindungsgemäßen Konjugats (entspr. Beispiel 3 und Figur 6) allein untersucht. Die Inkubationsdauer betrug 24 Stunden bei 5% CO₂ bei 37°C im Inkubator. Nach Entfernen des Medium-Mix und zweimaligem Waschen mit frischem RPMI-1640 wurde das gleiche Volumen frisches Medium zupipettiert und weiterkultiviert. Alle 72 Stunden erfolgte ein Mediumwechsel. Die Kulturen wurden wie zuvor behandelt: Wie im vorherigen Experiment wurde erneut bei etwa 72 Stunden ein Zusammenbrechen der unbehandelten Kultur festgestellt. Ungekoppelte Antisense-PNA und Zufallssequenz-NLS-PNA-Konjugate zeigten keinen Unterschied im Zellwachstum. Auffällig war bei der mit Antisense-NLS-PNA behandelten Kultur die relative Stabilität bis 144 Stunden nach Entzug von Antisense-NLS-PNA. Weiter wurde eine geringe Zunahme der Zellzahl bis zu einem Maximum bei 120 Stunden nach PNA-Entzug festgestellt.

## Patentansprüche

1. Konjugat zur spezifischen Hemmung der Expression eines *bcr*/*abl*-Fusionsgens, wobei das Konjugat die folgenden Komponenten aufweist:
(a) einen Transportvermittler für die Zellmembran;
(b) ein Adressprotein bzw. -peptid für den Import in den Zellkern; und
(c) eine mit dem Fusionsgen *bcr*/*abl* spezifisch hybridisierbare Peptid-Nukleinsäure (PNA), die dessen Expression hemmt.

2. Konjugat nach Anspruch 1, wobei der Transportvermittler ein Peptid oder Protein ist, das die Plasmamembran überwinden kann.

3. Konjugat nach Anspruch 1 oder 2, wobei : der Transportvermittler das Transmembranpeptid pAntp(43-58), TPU^{Eco}, TPU^{HIV-1/TAT} oder TPU^{Hum} ist.

4. Konjugat nach einem der Ansprüche 1 bis 3, wobei das Adressprotein bzw. -peptid ausgewählt ist aus: -Pro-Pro-Lys-Lys-Lys-Arg-Lys-Val-; und H₃N⁺-Pro-Lys-Lys-Lys-Arg-Lys-Val-.

5. Konjugat nach einem der Ansprüche 1 bis 4, wobei außerdem ein Spacer vorhanden ist.

6. Konjugat nach Anspruch 5, wobei sich der Spacer zwischen dem Adressprotein und der Peptid-Nukleinsäure. (PNA) befindet.

7. Konjugat nach Anspruch 6, wobei das Konjugat folgende Struktur hat: Transportvermittler-S-S-Adresspeptid-Spacer-PNA.

8. Konjugat oder Konjugat-Gemisch nach Anspruch 7, wobei die Peptid-Nukleinsäure die Sequenz TGC GTG TGG ATG ATT CTT TGT TTT AA umfaßt.

9. Arzneimittel, ein Konjugat nach einem der Ansprüche 1 bis 8 enthaltend.

10. Konjugat nach einem der Ansprüche 1 bis 8 zur Therapie der chronischen myeloischen Leukämie.

## Revendications

1. Conjugué pour l'inhibition spécifique de l'expression d'un gène de fusion bcr/abl, dans lequel le conjugué comprend les composés suivants :
a) un médiateur de transport pour la membrane cellulaire,
b) une protéine ou un peptide d'adressage pour l'importation dans le noyau de la cellule, et
c) un acide nucléique peptidique (PAN) spécifiquement hybridable avec le gène de fusion bcr/abl, acide qui inhibe son expression.

2. Conjugué selon la revendication 1, dans lequel le médiateur de transport est un peptide ou une protéine, apte à franchir la membrane du plasma.

3. Conjugué selon la revendication 1 ou 2, dans lequel le médiateur de transport est constitué par le peptide transmenbranique pAntp (43-58), TPU^{Eco}, TPU^{HIV-1/TAT} ou TPU^{Hum}.

4. Conjugué selon l'une des revendications 1 à 3, dans lequel la protéine ou le peptide d'adressage est choisi parmi -Pro-Pro-Lys-Lys-Lys-Arg-Lys-Val- ; et H₃N⁺-Pro-Lys-Lys-Lys-Arg-Lys-Val-.

5. Conjugué selon l'une des revendications 1 à 4, dans lequel un espaceur est en outre présent.

6. Conjugué selon la revendication 5, dans lequel l'espaceur se trouve entre la protéine d'adressage et l'acide nucléique peptidique (PAN).

7. Conjugué selon la revendication 6, dans lequel le conjugué présente la structure suivante : médiateur de transport-S-S-peptide d'adressage-espaceur-PAN.

8. Conjugué ou mélange de conjugué selon la revendication 7, dans lequel l'acide nucléique peptidique comprend les séquences TGC GTG TGG ATG ATT CTT TGT TTT AA.

9. Médicament comprenant un conjugué selon l'une des revendications 1 à 8.

10. Conjugué selon la revendication 1 à 8 appliqué à la thérapie des leucémie myéloïdes chroniques.

## Claims

1. A conjugate for specifically inhibiting the expression of a *bcr*/*abl* fusion gene, wherein the conjugate comprises the following components:
(a) a transport mediator for the cell membrane;
(b) an address protein or peptide for an importat into the nucleus; and
(c) a peptide nucleic acid (PNA) hybridizable specifically with the fusion gene *bcr*/*abl* and inhibiting the expression thereof.

2. The conjugate according to claim 1, wherein the transport mediator is a peptide or protein which can penetrate the plasma membrane.

3. The conjugate according to claim 1 or 2, wherein the transport mediator is the transmembrane peptide pAntp(43-58), TPU^{Eco}, TPU^{HIV-1/TAT} or TPU^{Hum}.

4. The conjugate according to any of claims 1 to 3, wherein the address protein or peptide is selected from:
-Pro-Pro-Lys-Lys-Lys-Arg-Lys-Val-; and
H₃N⁺-Pro-Lys-Lys-Lys-Arg-Lys-Val-.

5. The conjugate according to any of claims 1 to 4, wherein a spacer is additionally present.

6. The conjugate according to claim 5, wherein the spacer is located between address protein and peptide nucleic acid (PNA).

7. The conjugate according to claim 6, wherein the conjugate has the following structure: transport mediator-S-S-address peptide-spacer-PNA.

8. The conjugate or conjugate mixture according to claim 7, wherein the peptide nucleic acid comprises the sequence TGC GTG TGG ATG ATT CTT TGT TTT AA.

9. A medicament containing a conjugate according to any of claims 1 to 8.

10. The conjugate according to any of claims 1 to 8 for treating chronic myeloid leukemia.
